(19) Europäisches Patentamt
European Patent Office
Office européen des brevets

(11) Publication number: 0 248 107
B1

(12) # EUROPEAN PATENT SPECIFICATION

(45) Date of publication of patent specification: 18.07.90

(51) Int. Cl.$^5$: C 07 D 333/22, C 07 D 333/28, A 61 K 31/38

(21) Application number: 86112100.2

(22) Date of filing: 01.09.86

(54) **Thiophene ring-substituted alpha-(alkylaminopropionyl)-thiophene derivatives, a process for preparing them and pharmacuetical compositions containing them.**

(30) Priority: 03.06.86 ES 556311

(43) Date of publication of application:
09.12.87 Bulletin 87/50

(45) Publication of the grant of the patent:
18.07.90 Bulletin 90/29

(84) Designated Contracting States:
BE CH DE FR GB IT LI NL

(56) References cited:
EP-A- 196 001
FR-A-2 453 172
FR-M- 3 414
GB-A-1 313 150

(73) Proprietor: FERRER INTERNACIONAL, S.A.
Gran Via Carlos III, 94
08028 Barcelona (ES)

(72) Inventor: Foguet, Rafael
Llusanés 8
E-08022 Barcelona (ES)
Inventor: Forné, Ernesto
Felipe de Paz 15
E-08028 Barcelona (ES)
Inventor: Sacristán, Aurelio
Santa Amelia 2
E-08034 Barcelona (ES)
Inventor: Ortiz, José A.
Córcega 429
08037 Barcelona (ES)

(74) Representative: Kinzebach, Werner, Dr.
Patentanwälte Reitstötter, Kinzebach und Partner Sternwartstrasse 4 Postfach 86 06 49
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 248 107 B1

Courier Press, Leamington Spa, England.

## Description

The present invention relates to certain thiophene ring-substituted α-(alkylaminopropionyl)-thiophene derivatives, a process for preparing them and pharmaceutical composition containing them.

EP—A—196 001 which is a prior art document pursuant to article 54 (3) and (4) EPC relates to different thiophene ring-substituted α-(alkylaminopropionyl)-thiophene derivatives of the general formula (I):

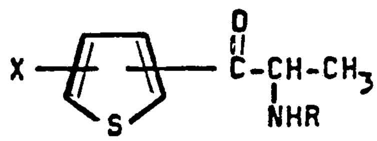

(I)

wherein X is a group different from hydrogen selected between methyl or chlorine and R is a linear or branched chain-alkyl group having 3 to 4 carbon atoms, and the nontoxic addition salts thereof, as well as to a process for preparing these compounds and to pharmaceutical compositions containing these compounds.

One object of the present invention relates to thiophene derivatives selected from:

2-[α-(sec-butylaminopropionyl)]-4-chlorothiophene,
2-[α-(sec-butylaminopropionyl)]-4-methylthiophene,
2-[α-(n-propylaminopropionyl)]-4-methylthiophene,
2-[α-(isopropylaminopropionyl)]-5-chlorothiophene,
2-[α-(n-propylaminopropionyl)]-5-chlorothiophene,
2-[α-(sec-butylaminopropionyl)]-5-chlorothiophene,
4-[α-(isopropylaminopropionyl)]-2-chlorothiophene,
4-[α-(sec-butylaminopropionyl)]-2-chlorothiophene

and the non-toxic acid addition salts thereof.

The preferred addition salts are the hydrochlorides.

The compounds of the present invention may be prepared according to the following scheme:

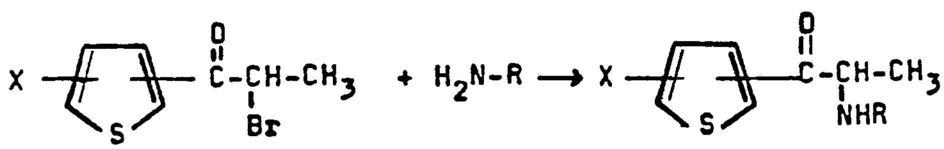

(II) (III) (I)

Bromine displacement in the compounds of formula II, wherein X is methyl or chlorine, by the amines of formula (III), wherein R is n-propyl, i-propyl or sec-butyl, occurs in an inert medium, preferably acetonitrile, and at a maximal temperature of 50°C, followed by vacuum evaporation of solvent, removal and evaporation to dryness of extracts. From the residue thus obtained, corresponding to the compounds of formula (I), it is possible to form their pharmaceutically acceptable salts, preferably hydrochlorides, by treating the respective acids in an inert solvent; ethers, specially diethyl ether, are preferred. Then the formed precipitate is separated by filtration.

α-(Bromopropionyl)-thiophene precursors of general formula (II) are obtained by conventional methods in Organic Chemistry; the reaction of propionyl-thiophenes with bromine in a medium composed by an aliphatic-chlorinated hydrocarbon, for example, methylene chloride, chloroform or carbon tetrachloride, are preferred. In turn, propionyl-thiophenes are susceptible to be obtained either by acylation of respective thiophenes with a derivative of propionic acid, preferably propionyl chloride, under suitable conditions of Friedel-Crafts' reaction, or by reacting lithium derivatives of respective thiophenes with propionitrile. When X is chlorine, starting compounds such as commercially-available chlorothiophenes or dichlorothiophenes are, if possible, used, and the substituent X = Cl may be introduced into the synthesis sequence on intermediate steps depending on the desired position. Sulphuryl chloride is preferred as a chlorinating agent. When X is methyl, respective methylthiophenes are, if possible, also used. The preparation of α-(bromopropionyl)-thiophene precursors of formula (II) is hereinafter illustrated.

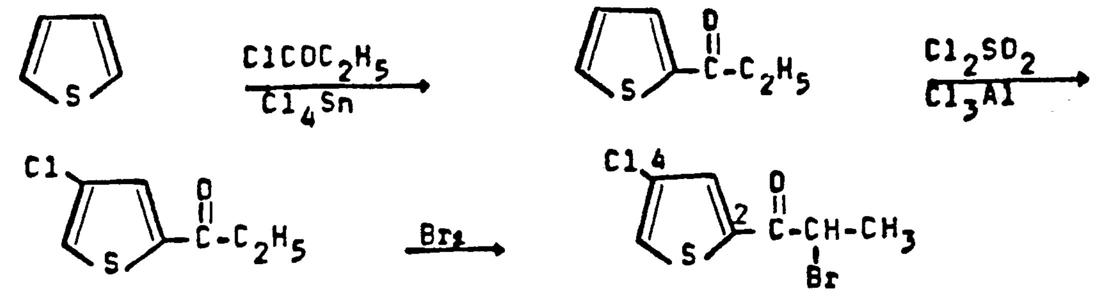


(II, X = 4-Cl; chain positions = 2)

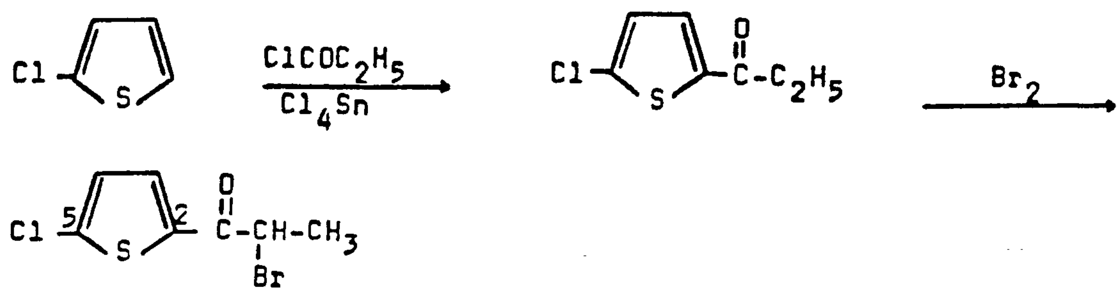


(II, X= 5-Cl; chain position= 2)

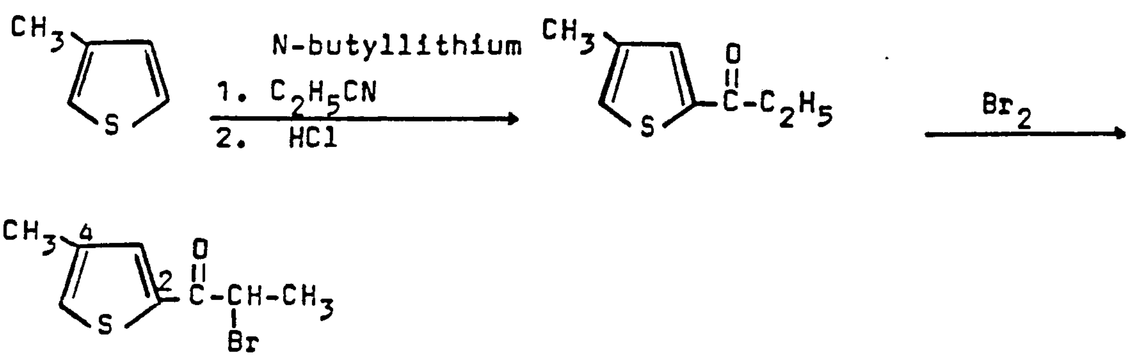


(II, X= 4-$CH_3$, chain position= 2)

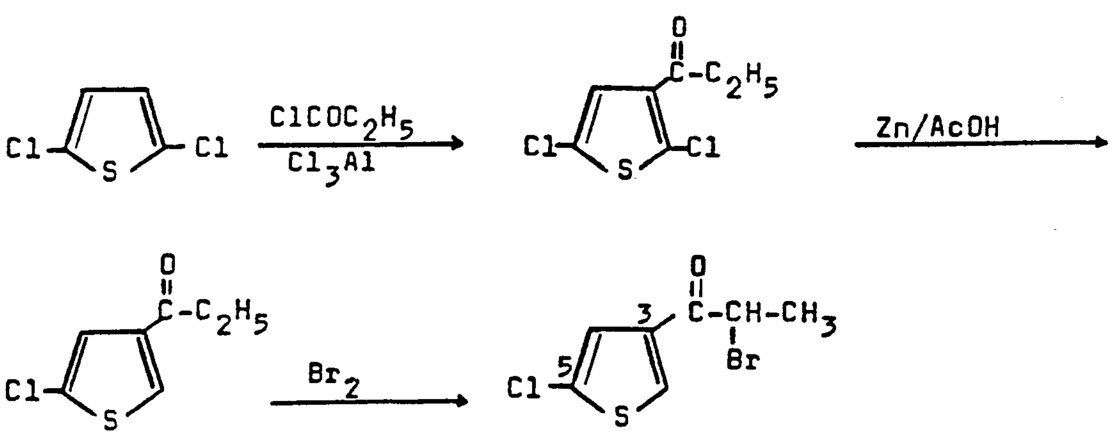


(II, X= 5-Cl, chain position= 3)

The compounds of the present invention have an effective antidepressant activity as evidenced by R. D. Porsolt (Immobility behavioural despair test in mice: Arch. Int. Pharmacodyn., 229, 327—336, 1977), which differ from the thiophene ring non-substituted α-(alkylaminopropionyl)-thiophenes, as disclosed in French Patent No. 3414M and British Patent No. 1313150, because these known compounds have anorexic activity devoided of central excitatory action or cardiovascular action, and neuroleptic, tranquillizing and analgesic action respectively. Consequently, the presence of an X group different from hydrogen, selected between methyl or chlorine, in the compounds of formula (I) leads to compounds having a very effective antidepressant action. Thus, the compounds of this invention are useful as medicament for treating depressions.

The compounds of the present invention mixed with pharmaceutically acceptable carriers can be administered by the oral route in the form of tablets, capsules, coated tablets, syrups, solutions, etc., by injectable route and by rectal route at daily doses ranging from 2.5 to 250 mg/kg.

By way of a non-limitative illustration within the essence of the invention, some examples are described hereinbelow referring to the possible way to obtain (I) by following the steps of the disclosed process.

Example 1

2-[α-(*sec*-butylaminopropionyl)]-4-chlorothiophene hydrochloride

To 26.05 g of α-bromo-4-chloro-2-propionylthiophene — prepared according to Spanish Patent No. 541.911 — in 30 ml of acetonitrile, 18.8 g of *sec*-butylamine are slowly added without exceeding 32°C. As the exothermia diminishes, the mixture is left under stirring at room temperature. The solvent is evaporated at vacuum (below 35°C), taken in methylene chloride (150 ml) and water (150 ml); the aqueous phase is removed with methylene chloride, the organic extracts are washed with water, dried and evaporated to dryness. The crude product (21.2 g) is dissolved in 400 ml of dry ethyl ether, cooled with water-ice bath and a dry HCl (g) stream is passed through; the insolubilized hydrochloride (20.0 g) is recrystallized from isopropanol to give 9.5 g (32%) of a white solid, m.p. 208—209°C and analysis correct.

IR Spectrum (KBr) $cm^{-1}$: 3200—2440, 1670, 1550, 1400, 1230, 1110, 1090, 860.

$^1$H—NMR ($d_6$-DMSO + $D_2O$) ppm: 0.87 (*t*, 3H, J=7Hz; $CH_3$—), 1.14 (*d*, 5H, J=6Hz; $CH_3$—CH—NH—), 1.47 (*d* + wide band, 5H, J=7Hz; $CH_3$— and —$CH_2$—), 2.87 (*m*, 1H; >*CH*—NH—), 4.48 (*m*, 1H; >*CH*—$CH_3$), 8.11 (*s*, 1H; H-thiophene) and 8.26 (*s*, 1H; H-thiophene).

Example 2

2-[α-(*sec*-butylaminopropionyl)]-4-methylthiophene hydrochloride

From 33.9 g of α-bromo-4-methyl-2-propionylthiophene — prepared according to Spanish Patent No. 552.220 — 26.6 g of *sec*-butylamine in 45 ml of acetonitrile, and operating as described in Ex. 1, 41.9 g of crude product are isolated, respective hydrochloride (23.1 g) formed in ethyl ether is recrystallized in absolute ethanol to give 12.4 g (32%) of a white solid, m.p. 227—229°C and analysis correct.

IR Spectrum (KBr) $cm^{-1}$: 3200—2400, 1665, 1545, 1410, 1225, 1100, 885, 860.

$^1$H—NMR Spectrum ($d_6$-DMSO) ppm: 0.90 (*t*, 3H, J=7Hz; $CH_3$—), 1.25 (*d*, 3H, J=7Hz; $CH_3$—CN NH—), 1.57 (*d* + wide band, 5H, J=7Hz; $CH_3$— and $CH_2$—), 2.26 (*s*, 3H; $CH_3$-thiophene), 3.00 (*m*, 1H; >*CH*—NH—), 5.10 (*m*, 1H; >*CH*—$CH_3$), 7.80 (*s*, 1H; H-thiophene) and 8.20 (*s*, 1H; H-thiophene).

Example 3

2-[α-(*n*-propylaminopropionyl)]-4-methylthiophene hydrochloride

From 33.9 g of α-bromo-4-methyl-2-propionylthiophene in 45 ml of acetonitrile and 21.5 g of n-propylamine, and operating as described in Ex. 1, 42.1 g of crude product are isolated, respective hydrochloride (26.1 g) formed in ethyl ether is recrystallized in acetonitrile-isopropanol (1:1) to give 11.8 g (33%) of a white solid, m.p. 211.3—212°C and analysis correct.

IR Spectrum (KBr) $cm^{-1}$: 3200—2400, 1660, 1410, 1230, 1215, 1105, 920, 780.

$^1$H—NMR ($d_6$-DMSO) ppm: 0.90 (*t*, 3H, J=7Hz; $CH_3$—), 1.56 (*d*, 3H, J=7Hz; $CH_3$—CH—CO—), 1.74 (*m*, 2H, J=8Hz; —$CH_2$—), 2.27 (*s*, 3H; $CH_3$-thiophene), 2.82 (*m*, 2H, J=8Hz; —$CH_2$—NH—), 5.00 (*q*, 1H, J=7Hz; >CH—CO—), 7.81 (*s*, 1H; thiophene) and 8.07 (*s*, 1H) H-thiophene).

Example 4

2-[α-(*iso*propylaminopropionyl)]-5-chlorothiophene hydrochloride

From 32.0 g of α-bromo-5-chloro-2-propionylthiophene — prepared as described in Spanish Patent No. 541.911 — in 40 ml of acetonitrile and 18.62 g of isopropylamine, and operating as described in Exa. 1, 30 g of crude product are isolated, respective hydrochloride (28.7 g) formed in ethyl ether is recrystallized from isopropanol to give 13.5 g (39%) of a white solid, m.p. 222.2—223°C and analysis correct.

IR Spectrum (KBr) $cm^{-1}$: 3160—2400, 1660, 1555, 1410, 1320, 1240, 1075, 1010, 825.

$^1$H—NMR ($d_6$-DMSO) ppm: 1.30 (*d*, 6H, J=6Hz; —$CH(CH_3)_2$), 1.57 (*d*, 3H, J=7Hz; $CH_3$—), 3.37 (*m*, 1H; *CH*$(CH_3)_2$), 5.17 (*m*, 1H; —CO—CH—), 7.42 (*d*, 1H, J=4Hz; H-thiophene) and 8.37 (*d*, 1H, J=4Hz; H-thiophene).

Example 5

2-[α-(*n*-propylaminopropionyl)]-5-chlorothiophene hydrochloride

From 35.7 g of α-bromo-5-chloro-2-propionylthiophene in 45 ml of acetonitrile and 20.9 g of n-propylamine, and operating as described in Ex. 1, 31 g of crude product are isolated, respective hydrochloride (12 g) is recrystallized from isopropanol to give 7.73 g (20%) of a white solid, m.p. 190—190.3°C and analysis correct.

IR Spectrum (KBr) $cm^{-1}$: 3180—2400, 1665, 1410, 1320, 1230, 1010, 815, 720.

$^1H$—NMR Spectrum ($D_2O$) ppm: 1.00 (*t*, 3H, J=7.5Hz; $CH_3$—), 1.68 (*d*, 3H, J=7Hz; $CH_3$—CH—CO—), 1.82 (*m*, 2H; —$CH_2$—), 3.10 (*m*, 2H; —$CH_2$—N<), 5.00 (*q*, 1H, J=7.0Hz; >CH—CO—), 7.25 (*d*, 1H, J=4Hz, H-thiophene) and 7.94 (*d*, 1H, J=4Hz; H-thiophene).

Example 6

2-[α-(*sec*-butylaminopropionyl)]-5-chlorothiophene hydrochloride

From 32.9 g of α-bromo-5-chloro-2-propionylthiophene in 40 ml of acetonitrile and 23.8 g of *sec*-butylamine, and operating as described in Ex. 1, 28.4 g of crude product are isolated, respective hydrochloride (20.5 g) is recrystallized from isopropanol to give 9.2 g (26%) of a white solid, m.p. 191.3—192°C and analysis correct.

IR Spectrum (KBr) $cm^{-1}$: 3120—2400, 1660, 1550, 1420, 1235, 1010, 820.

$^1H$—NMR ($d_6$-DMSO + $D_2O$) ppm: 0.90 (2*t*, 3H, J=7Hz; $CH_3$—), 1.27 (*d*, 3H, J=7Hz; $CH_3$—CH—NH—), 1.59 (*d* + wide band, 5H, J=7Hz; $CH_3$— and —$CH_2$—), 3.05 (*m*, 1H; >*CH*—NH), 5.15 (*qt*, 1H, J=7Hz; >CH—CO—), 7.40 (*d*, 1H, J=4Hz; H-thiophene) and 8.32 (*d*, 1H, J=4Hz; H-thiophene).

Example 7

4-[α-(*iso*propylaminopropionyl)]-2-chlorothiophene hydrochloride

From 33.9 g of α-bromo-2-chloro-4-propionylthiophene — prepared according to Spanish Patent No. 541.911 — in 40 ml of acetonitrile ankd 19.8 g of isopropylamine, and operating as described in Ex. 1, 31.0 g of crude product are isolated, respective hydrochloride (28.5 g) formed in ethyl ether is recrystallized in ethanol to give 9.3 g (30%) of a white solid, m.p. 244—245°C and analysis correct.

IR Spectrum (KBr) $cm^{-1}$: 3200—2420, 1670, 1550, 1455, 1420, 1210, 1190, 1170, 1100, 1000, 830.

$^1H$—NMR ($D_2O$) ppm: 1.35 (*d*, 6H, J=6Hz; —CH($CH_3$)$_2$), 1.62 (*d*, 3H, J=7Hz; $CH_3$—), 3.50 (*m*, 1H, J=7Hz; *CH*($CH_3$)$_2$), 5.00 (*q*, 1H, J=7Hz; —CO—CH—), 7.52 (*d*, 1H, J=2Hz; H-thiophene) and 8.44 (*d*, 1H, J=2Hz; H-thiophene).

Example 8

4-[α-(*sec*-butylaminopropionyl)]-2-chlorothiophene hydrochloride

From 32.2 g of α-bromo-2-chloro-4-propionylthiophene in 40 ml of acetonitrile and 23.5 g of *sec*-butylamine, and operating as described in Ex. 1, 30 g of crude product are isolated, respective hydrochloride (20 g) formed in ethyl ether is recrystallized in isopropanol to give 6.55 g (21%) of a white solid, m.p. 206—207°C and analysis correct.

IR Spectrum (KBr) $cm^{-1}$: 3160—2400, 1680, 1550, 1420, 1210, 1195, 1105, 1005, 870, 805.

$^1H$—NMR Spectrum ($CD_3OD$) ppm: 1.00 (2*t*, 3H, J=7.0Hz; $CH_3$—$CH_2$—), 1.38 (*d*, 3H, J=6Hz; $CH_3$—CH—NH—), 1.63 (*d* + wide band, 5H, J=7Hz; $CH_3$ and —$CH_2$), 3.14 (*m*, 1H; >CH—NH—), 5.12 (*m*, 1H; >CH—CO—), 7.53 (*d*, 1H, J=2Hz; H-thiophene) and 8.60 (*d*, 1H, J=2Hz; H-thiophene).

**Claims**

1. Substituted α-(alkylaminopropionyl)-thiophene derivatives selected from:
2-[α-(sec-butylaminopropionyl)]-4-chlorothiophene,
2-[α-(sec-butylaminopropionyl)]-4-methylthiophene,
2-[α-(n-propylaminopropionyl)]-4-methylthiophene,
2-[α-(isopropylaminopropionyl)]-5-chlorothiophene,
2-[α-(n-propylaminopropionyl)]-5-chlorothiophene,
2-[α-(sec-butylaminopropionyl)]-5-chlorothiophene,
4-[α-(isopropylaminopropionyl)]-2-chlorothiophene,
4-[α-(sec-butylaminopropionyl)]-2-chlorothiophene
and the non-toxic acid addition salts thereof.

2. A process for preparing the compounds according to claim 1, characterized in that an (α-bromopropionyl)-thiophene of the general formula (II):

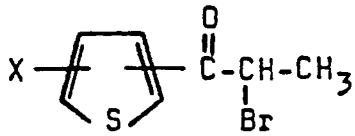

(II)

wherein X is methyl or chlorine
is reacted with an amine of the general formula (III):

$$H_2N—R \qquad (III)$$

wherein R is n-propyl, i-propyl or sec-butyl, in an inert medium, and at a maximal temperature of 50°C and, if desired, the free base is treated with an acid to obtain a nontoxic addition salt.

3. The process of Claim 2, characterized in that acetonitrile is used as inert medium in the reaction between (II) and (III).

4. The process of Claim 2, characterized in that an ether is used as inert medium in the formation of the nontoxic addition salts.

5. The process of Claim 2, characterized in that the ether is diethyl ether.

6. Pharmaceutical compositions comprising at least one of the compounds of Claim 1, optionally together with pharmaceutical carriers and/or adjuvants.

7. The use of the compounds according to Claim 1 for the preparation of pharmaceutical compositions for treating depressions.

**Patentansprüche**

1. Substituierte α-(Alkylaminopropionyl)-thiophen Derivative ausgewählt unter:
2-[α-(sek-Butylaminopropionyl)]-4-chlorthiophen,
2-[α-(sek-Butylaminopropionyl)]-4-methylthiophen,
2-[α-(n-Propylaminopropionyl)]-4-methylthiophen,
2-[α-(Isopropylaminopropionyl)]-5-chlorthiophen,
2-[α-(n-Propylaminopropionyl)]-5-chlorthiophen,
2-[α-(sek-Butylaminopropionyl)]-5-chlorthiophen,
4-[α-(Isopropylaminopropionyl)]-2-chlorthiophen,
4-[α-(sek-Butylaminopropionyl)]-2-chlorthiophen
und die nicht-toxischen Säureadditionssalze davon.

2. Verfahren zur Herstellung der Verbindungen gemäß Anspruch 1, dadurch gekennzeichnet, daß ein (α-Brompropionyl)thiophen der allgemeinen Formel II:

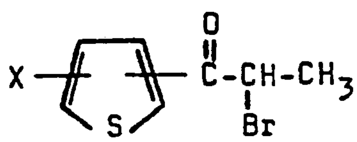

(II)

worin X für Methyl oder Chlor steht,
mit einem Amin der allgemeinen Formel III:

$$H_2N—R \qquad (III)$$

worin R für n-Propyl, i-Propyl oder sek-Butyl steht, in einem inerten Medium und bei einer Maximaltemperatur von 50°C umgesetzt und falls gewünscht, die freie Base mit einer Säure behandelt wird, um ein nicht-toxisches Additionssalz zu erhalten.

3. Verfahren nach Anspruch 2, dadurch gekennzeichnet, daß Acetonitril als inertes Medium für die Reaktion zwischen II und III verwendet wird.

4. Verfahren nach Anspruch 2, dadurch gekennzeichnet, daß ein Äther als inertes Medium bei der Bildung der nicht-toxischen Additionssalze verwendet wird.

5. Verfahren nach Anspruch 2, dadurch gekennzeichnet, daß der Äther Diäthyläther ist.

6. Pharmazeutische Zusammensetzungen mit zumindest einer Verbindung nach Anspruch 1,

gegebenenfalls mit pharmazeutischen Trägern und/oder zusätzlichen Adjuvantien.

7. Verwendung der Verbindungen nach Anspruch 1 zur Herstellung von pharmazeutischen Zusammensetzungen zur Behandlung von Depressionen.

**Revendications**

1. Dérivés substitués de l'α-(alkylaminopropionyl)-thiophènes choisis parmi:
le 2-[α-(sec-butylaminopropionyl)]-4-chlorothiophène,
le 2-[α-(sec-butylaminopropionyl)]-4-méthylthiophène,
le 2-[α-(n-propylaminopropionyl)]-4-méthylthiophène,
le 2-[α-(isopropylaminopropionyl)]-5-chlorothiophène,
le 2-[α-(n-propylaminopropionyl)]-5-chlorothiophène,
le 2-[α-(sec-butylaminopropionyl)]-5-chlorothiophène,
le 4-[α-(isopropylaminopropionyl)]-2-chlorothiophène,
le 4-[α-(sec-butylaminopropionyl)]-2-chlorothiophène
et leurs sels d'addition aux acides non toxiques.

2. Procédé de préparation des composés suivant la revendication 1, caractérisé en ce qu'on fait réagir un (α-bromopropionyl)-thiophène répondant à la formule générale (II):

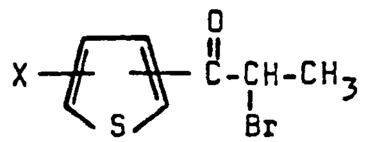

(II)

dans laquelle X est un groupe méthyle ou un atome de chlore
avec une amine répondant à la formule générale (III):

$$H_2N—R \qquad (III)$$

dans laquelle R est un groupe n-propyle, i-propyle ou sec-butyle, dans un milieu inerte, et à une température maxima de 50°C et, si on le désire, en ce qu'on traite la base libre par un acide pour obtenir un sel d'addition non toxique.

3. Procédé selon la revendication 2, caractérisé en ce que l'on utilise comme milieu inerte l'acétonitrile dans la réaction entre (II) et (III).

4. Procédé selon la revendication 2, caractérisé en ce qu'on utilise un éther comme milieu inerte dans la formation des sels d'addition non toxiques.

5. Procédé selon la revendication 2, caractérisé en ce que léther est l'éther diéthylique.

6. Compositions pharmaceutiques comprenant au moins un des composés de la revendication 1 avec, si on le désire, des supports et/ou adjuvants pharmaceutiques.

7. Utilisation des composés selon la revendication 1 pour la préparation de compositions pharmaceutiques pour le traitement des dépressions.